# FASCICULE DE BREVET EUROPEEN

(11) **EP 1 560 826 B1**
(45) Date de publication et mention de la délivrance du brevet: **12.04.2006**
(21) Numéro de dépôt: 03767886.9
(22) Date de dépôt: 04.11.2003
(51) Int. Cl.: C07D 471/04, A61K 31/505

(54) **COMPOSES PYRIDOPYRIMIDINONE, LEUR PROCEDE DE PREPARATION ET LES COMPOSITIONS PHARMACEUTIQUES QUI LES CONTIENNENT**
PYRIDOPYRIMIDINON-DERIVATE, VERFAHREN ZU IHRER HERSTELLUNG UND DIESE ENTHALTENDE PHARMAZEUTISCHE ZUSAMMENSETZUNGEN
PYRIDOPYRIMIDINONE COMPOUNDS, METHOD FOR PRODUCTION THEREOF AND MEDICAMENTS COMPRISING THE SAME

(30) Priorité: 05.11.2002 FR 0213804
(43) Date de publication de la demande: 10.08.2005
(73) Titulaire: Les Laboratoires Servier, 92415 Courbevoie Cedex (FR)
(72) Inventeur: RAULT, Sylvain, F-14370 Moult (FR); LANCELOT, Jean-Charles, F-14400 Le Bourg (FR); KOPP, Marina, F-14000 Caen (FR); CAIGNARD, Daniel-Henri, F-78230 Le Pecq (FR); PFEIFFER, Buno, F-95320 Saint Leu la Forêt (FR); RENARD, Pierre, F-78150 Le Chesnay (FR)
(86) Numéro de dépôt international: PCT/FR2003/003274
(87) Numéro de publication internationale: WO 2004/043956

(56) Documents cités:
- WO-A-98/02438
- WO-A-98/05661
- US-A- 6 060 477
- US-B1- 6 313 292
- J.H. MAGUIRE: "The Synthesis of a Pyrido[3,4-d]pyrimidine Analog of Pteroic Acid" J. HET. CHEM., vol. 16, 1979, pages 133-136, XP002233960

## Description

La présente invention concerne de nouveaux dérivés pyridopyrimidinone, leur procédé de préparation et les compositions pharmaceutiques qui les contiennent.

Les composés de l'invention sont nouveaux et présentent d'intéressantes propriétés modulatrices d'un panel de kinases ce qui les rend utiles dans le traitement de nombreux types d'affections parmi lesquelles on peut citer à titre non limitatif: cancer, arthrose, diabète, obésité, hypertension etc. Ils sont en outre totalement atoxiques. Par ailleurs, à notre connaissance, cette famille de composés est totalement originale et l'activité que nous lui avons découverte n'a pas été mentionnée pour des dérivés structuralement proches.

Le brevet US 6,060,477 décrit des dérivés de pyrimidinone condensés en tant que modulateurs de l'apoptose.

Plus particulièrement la présente invention concerne les composés de formule (I) : 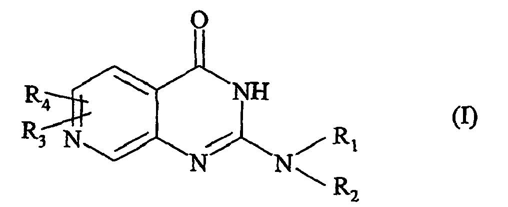 dans laquelle :
➢ R₁ et R₂, identiques ou différents, représentent un atome d'hydrogène, un groupement alkyle ou forment ensemble avec l'atome d'azote qui les porte un hétérocycle,
➢ R₃ représente un atome d'halogène, un groupement alkoxy, un groupement aryle éventuellement substitué, ou un groupement NR'₁R'₂ avec R'₁ et R'₂, identiques ou différents, représentant un atome d'hydrogène, un groupement alkyle ou formant ensemble avec l'atome d'azote qui les porte un hétérocycle,
➢ R₄ représente un atome d'hydrogène ou un groupement N R"₁ R"₂ avec R"₁ et R"₂, identiques ou différents, représentant un atome d'hydrogène, un groupement alkyle ou formant ensemble avec l'atome d'azote qui les porte un hétérocycle,
leur énantiomères, diastéréoisomères, tautomères ainsi que leurs sels d'addition à un acide
ou à une base pharmaceutiquement acceptable,
étant entendu que :
- le terme alkyle désigne une chaîne hydrocarbonée, linéaire ou ramifiée, comprenant de 1 à 8 atomes de carbone,
- le terme alkoxy désigne un groupement alkyle oxy dont la chaîne alkyle, linéaire ou ramifiée, contient de 1 à 8 atomes de carbone,
- le terme aryle désigne un groupement phényle ou naphthyle,
- le terme hétérocycle désigne un système mono ou bicyclique, comprenant de 5 à 11 atomes de carbone, et pouvant contenir outre l'atome d'azote auquel sont liés R₁ R₂, R'₁ R'₂, ou R"₁ R"₂ un ou deux autres hétéroatomes choisis parmi oxygène, soufre et azote, ce système hétérocyclique pouvant être substitué par un, deux, ou trois groupements alkyle,
- le terme substitué affectant le groupement aryle signifie que les groupements phényle ou naphthyle sont substitués par un, deux, ou trois groupements, identiques ou différents, choisis parmi les atomes d'halogène, les groupements alkyle, alkoxy, polyhalogénoalkyle, et hydroxy, étant entendu que par polyhalogénoalkyle on entend une chaîne carbonée, linéaire ou ramifiée, contenant de 1 à 3 atomes de carbone et de 1 à 7 atomes d'halogène.

Parmi les acides pharmaceutiquement acceptables, on peut citer à titre non limitatif, les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthane sulfonique, camphorique etc....

Parmi les bases pharmaceutiquement acceptables on peut citer à titre non limitatif l'hydroxyde de sodium, l'hydroxyde de potassium, la triéthylamine, etc....

Un aspect avantageux de l'invention concerne les composés de formule (I') : 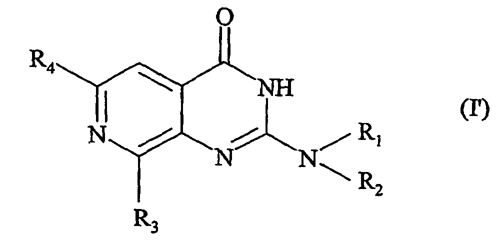 leurs énantiomères, diastéréoisomères tautomères ainsi que leurs sels d'addition à un acide ou à une base pharmaceutiquement acceptable.

Un autre aspect avantageux de l'invention concerne les composés pour lesquels NR₁R₂ représente un groupement NH₂, un groupement di *n*-propylamine ou encore un groupement morpholine, leurs énantiomères, diastéréoisomères, tautomères ainsi que leurs sels d'addition à un acide ou une base pharmaceutiquement acceptable.

Des composés préférés de l'invention sont ceux pour lesquels R₃ représente un groupement 3,4-diméthoxyphényle, 3,5-diméthylmorpholine, thiomorpholine, azépine, perhydroquinoline, ou pyrrolidine, ou un atome de chlore, leur énantiomères, diastéréoisomères, tautomères ainsi que leurs sels d'addition à un acide ou une base pharmaceutiquement acceptable.

Un autre aspect avantageux de l'invention concerne les dérivés pour lesquels R₄ représente un atome d'hydrogène, un groupement morpholine, ou azépine, leurs énantiomères, diastéréoisomères, tautomères ainsi que leurs sels d'addition à un acide ou à une base pharmaceutiquement acceptable.

Parmi les composés préférés de l'invention, on peut citer :
la 2-(dipropylamino)-8-(4-thiomorpholinyl)pyrido[3,4-*d*]pyrimidin-4(3*H*)-one,
la 8-(1-azocanyl)-2-(dipropylamino)pyrido[3,4-*d*]pyrimidin-4(3*H*)-one,
la 8-((4a*α*,8a*α*)-octahydro-1(2*H*-quinolinyl)-2-(dipropylamino)pyrido[3,4-*d*]pyrimidin-4(3*H*)-one,
la 8-((4a*β*,8a*α*)-octahydro-1(2*H*)-quinolinyl)-2-(dipropylamino)pyrido[3,4-*d*]pyrimidin-4(3*H*)-one,
la 6,8-di(1-azépanyl)-2-(dipropylamino)pyrido[3,4-*d*]pyrimidin-4(3*H*)-one,
la 8-(1-azépanyl)-2-(dipropylamino)-6-(4-morpholinyl)pyrido[3,4-*d*]pyrimidin-4(3*H*)-one,
la 8-(1-azépanyl)-2,6-di(4-morpholinyl)pyrido[3,4-*d*]pyrimidin-4(3*H*)-one,
la 2-amino-8-[(3*α*,5*β*)-3,5-diméthylinorpholinyl]pyrido[3,4-*d*]pyrimidin-4(3*H*)-one,
la 2-amino-8-[(3*α*,5*α*)-3,5-diméthylmorpholinyl]pyrido[3,4-*d*]pyrimidin-4(3*H*)-one,
la 8-[(3*α*,5*β*)-3,5-diméthylmorpholinyl]-2-(dipropylamino)pyrido[3,4-*d*]pyrimidin-4(3*H*)-one,
la 8-[(3*α*,5*α*)-3,5-diméthylmorpholinyl]-2-(dipropylamino)pyrido[3,4-*d*]pyrimidin-4(3*H*)-one,
la 8-[(3*α*,5*α*)-3,5-diméthylmorpholinyl]-2-(4-morpholinyl)pyrido[3,4-*d*]pyrimidin-4(3*H*)-one,
la 2-amino-8-(1-azépanyl)-6-(4-morpholinyl)pyrido[3,4-*d*]pyrimidin-4(3*H*)-one,
la 8-chloro-2-(dipropylamino)pyrido[3,4-*d*]pyrimidin-4(3*H*)-one,
la 2-(dipropylamino)-8-(1-pyrrolidinyl)pyrido[3,4-*d*]pyrimidin-4(3*H*)-one,
et la 8-(3,4-diméthoxyphényl)-2-(dipropylamino)pyrido[3,4-*d*]pyrimidin-4(3*H*)-one,
leurs tautomères et leurs sels d'addition à un acide pharmaceutiquement acceptable.

L'invention concerne également le procédé de préparation des composés de formule (I) caractérisé en ce que l'on utilise comme produit de départ les composés de formule (II) : 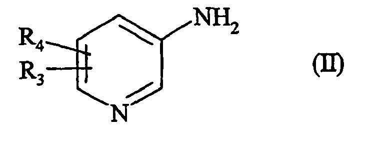 dans laquelle :
R₃ et R₄ ont la même définition que dans la formule (I), que l'on condense avec un dérivé de formule (III) :

   S=C=N-C(O)OR₂₀ (III)

   avec R₂₀ représentant un groupement alkyle ou arylalkyle,
   pour conduire à un dérivé de formule (IV) : 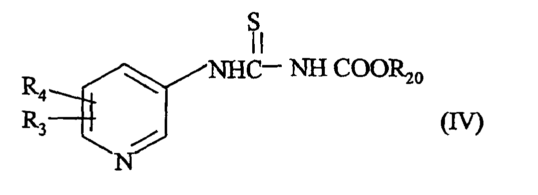
dans laquelle :
R₃, R₄ et R₂₀ ont la même signification que précédemment, dérivé de formule (IV), qui se condense en présence d'un sel métallique avec l'amine (V) :

   HN R₁ R₂ (V)
dans laquelle :
R₁ et R₂ ont la même signification que dans la formule (I), pour conduire à un composé de formule (I),
   - qui peut-être, le cas échéant, purifié selon une technique classique de purification,
   - dont on sépare, le cas échéant, les stéréoisomères selon une technique classique de séparation,
   - que l'on transforme, si l'on le souhaite, en ses sels d'addition à un acide ou une base pharmaceutiquement acceptable,
   étant entendu :
   - qu'à tout moment jugé opportun au cours du procédé précédemment décrit, le ou les groupements, amino, alkylamino des réactifs de départ (II) peuvent être protégés puis, après condensation, déprotégés pour les besoins de la synthèse,
   - que les réactifs (II), sont décrits dans la littérature ou sont préparés par des modes opératoires connus, décrits dans la littérature.

Les composés de l'invention ont été étudiés sur un ensemble Kinases sur lesquelles ils ont montré une excellente activité. Cette activité est généralement sélective pour un type de Kinase considérée, type qui varie en fonction de la structure du produit de formule (I). En fonction du type de Kinase sur laquelle le composé de l'invention est actif, on peut en attendre une excellente activité dans divers types de cancers, dans les troubles du métabolisme et plus particulièrement dans le traitement ou la prophylaxie des hyperglycémies, dyslipidémies, telles que hypercholestérolémie, hyperlipidémie et également dans le traitement des diabètes non insulino dépendants de type II, de l'obésité, des complications diabétiques en particulier au niveau cardiovasculaire, ou encore dans des troubles inflammatoires tels que l'arthrose, ou enfin dans des troubles cardiovasculaires non associés au diabète comme l'hypertension artérielle. Le fait par ailleurs que les composés de l'invention soient totalement atoxiques leur confère un indéniable intérêt en thérapeutique.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie varie selon l'âge et le poids du patient, la nature et la sévérité de l'affection ainsi que la voie d'administration. Celle-ci peut-être orale, nasale, rectale ou parentérale. D'une manière générale, la posologie unitaire s'échelonne entre 0,01 et 500 mg pour un traitement en 1 à 3 prises par 24 heures.

Les exemples suivants illustrent l'invention et ne la limitent en aucune façon. Les structures des composés décrits ont été confirmées par des techniques spectroscopiques et spectrométriques usuelles.

Les produits de départ utilisés sont des produits connus ou préparés selon des modes opératoires connus.

Par composé (4a*α*,8a*α*), on entend composé dont la jonction de cycle correspondante est de configuration cis.

Par composé (3*α*,5*α*), on entend composé dont la jonction de cycle correspondante est de configuration cis.

Par composé (4a*β*,8a*α*), on entend composé dont la jonction correspondante de cycle est trans.

Par composé (3*α*,5*β*), on entend composé dont la jonction correspondante de cycle est trans.

### Exemple 1 :

### 8-(1-Azocanyl)-2-(dipropylamino)pyrido[3,4-d]pyrimidin-4(3H)-one

Un mélange de 0,02 mole (4,10 g) de 3-amino-2-azocanopyridine et de 0,02 mole d'isothiocyanate d'éthoxycarbonyle est agité à température ambiante pendant 3 heures dans 100 ml de DMF ; on obtient ainsi la thiourée de formule (IV) qu'il n'est pas nécessaire d'isoler.
La solution est refroidie à 0°C et est saturée par 2,5 eq de di *n*-propylamine et on ajoute 0,02 mole de chlorure mercurique ; au bout de 15 minutes, le bain de glace est enlevé et le mélange est agité à température ambiante pendant 3 heures (on observe toujours l'apparition du précipité noir dû à la formation de sulfure de mercure). Après avoir ajouté 150 ml d'acétate d'éthyle, la solution est filtrée sur célite et les solvants sont évaporés sous pression réduite. La *N*-éthoxycarbonylguanidine brute, ainsi obtenue est de nouveau dissoute dans 50 ml de DMF et chauffée à reflux pendant 2 heures. Après avoir filtré à chaud la solution afin d'éliminer les derniers restes de sel de mercure, la DMF est évaporée sous pression réduite. Le solide ainsi obtenu est repris dans un minimum d'acétonitrile, essoré sur fritté puis recristallisé dans d'acétonitrile.
Point de fusion : 172°C

### Exemple 2 :

### 8-(4-Thiomorpholinyl)-2-(dipropylamino)pyrido[3,4-d]pyrimidin-4(3H)-one

En procédant comme dans l'exemple 1 mais en remplaçant la 3-amino-2-azocanopyridine par la 3-Amino-2-(4-thiomorpholinyl) pyridine, on obtient le produit du titre.
Point de fusion : 226°C

### Exemple 3 :

### 8-((4aα,8aα)-Octahydro-1(2H)-quinoliny)-2-(dipropylamino)pyrido[3,4-d]pyrimidin-4(3H)-one

En procédant comme dans l'exemple 1 mais en remplaçant la 3-amino-2-azocanopyridine par la 2-((4aα,8aα)-octahydro-1(2*H*)-quinolinyl)-3-pyridinamine, on obtient le produit du titre.
Point de fusion : 215°C

### Exemple 4 :

### 8-((4aβ,8aα)-Octahydro-1(2H)-quinolinyl)-2-(dipropylamino)pyrido[3,4-d]pyrimidin-4(3H)-one

En procédant comme dans l'exemple 1 mais en remplaçant la 3-amino-2-azocanopyridine par la 2-((4aβ,8aα)-octahydro-1(2*H*)-quinolinyl)-3-pyridinamine, on obtient le produit du titre.
Point de fusion : 225°C

### Exemple 5 :

### 6,8-Di(1-azépanyl)-2-(dipropylamino)pyrido[3,4-d]pyrimidin-4(3H)-one

En procédant comme dans l'exemple 1, mais en remplaçant la 3-amino-2-azocanopyridine par la 3-amino-2,6-diazépanopyridine, on obtient le produit du titre.
Point de fusion : 220°C

### Exemple 6 :

### 8-(1-Azépanyl)-2-(dipropylamino)-6-(4-morpholinyl)pyrido[3,4-d]pyrimidin-4(3H)-one

En procédant comme dans l'exemple 1 mais en remplaçant la 3-amino-2-azocanopyridine par la 3-amino-2-azépano-6-(4-morpholinyl)pyridine, on obtient le produit du titre.
Point de fusion : 250°C

### Exemple 7 :

### 8-(1-Azépanyl)2,6-di(4-morpholinyl)pyrido[3,4-d]pyrimidin-4(3H)-one

En procédant comme dans l'exemple 6 mais en remplaçant la di *n*-propylamine par la morpholine, on obtient le produit du titre.
Point de fusion : 240°C

### Exemple 8 :

### 2-Amino-8-[(3α,5β)-3,5-diméthylmorpholinyl]pyrido[3,4-d]pyrimidin-4(3H)-one

### Stade A : N-éthoxycarbonyl-N'-2 (3,5-diméthymorpholin-4-yl) pyridine

Un mélange de 0,02 mole de cis / trans 3-amino 2-[4-(3,5 diméthyl) morpholinyl] pyridine (3,80g) et de 2,62g (0,02 mole) isothiocyanate d'éthoxycarbonyle est agité à température ambiante pendant 3 heures dans 100 ml de DMF. Le mélange obtenu est versé dans 200 ml d'eau. Le précipité qui se forme est essoré et lavé à l'éther de pétrole. Les isomères cis et trans sont séparés par chromatographie sur colonne (éluant éther / cyclohexane 55/45).

### Stade B : 2-Amino-8-[(3α,5β)-3,5-diméthylmorpholinyl]pyrido[3,4-d]pyrimidin-4(3H)-one

0,002 mole de la cis thiourée obtenue au stade A est dissoute dans 100 ml de DMF et la solution ainsi formée est refroidie à 0°C puis saturée par l'ammoniac gazeux. On ajoute 0,002 mole (0,50g) de chlorure mercurique. Au bout de quinze minutes, le bain de glace est enlevé et le mélange agité à température ambiante pendant 3 heures. Après addition de 150 ml d'acétate d'éthyle, la solution est filtrée sur célite et les solvants évaporés sous pression réduite. Le précipité ainsi obtenu est à nouveau dissous dans 50 ml de DMF et chauffé à reflux pendant deux heures. Après filtration à chaud, la DMF est évaporée sous pression réduite. Le solide ainsi obtenu est repris dans un minimum d'acétonitrile, essoré sur fritté et recristallisé dans l'acétonitrile.
Point de fusion supérieure à 260°C

### Exemple 9 :

### 2-Amino-8-[(3α,5α)-3,5-diméthylmorpholinyl]pyrido[3,4-d]pyrimidin-4(3H)-one

En utilisant la trans *N*-éthoxycarbonyl-*N*'-2-[3-(3,5-diméthyl)morpholin-4yl)-pyridin-thiourée obtenue stade A exemple 8 et en procédant comme dans l'exemple 8 stade B on obtient le produit du titre.

### Exemple 10 :

### 8-[(3α,5α)-3,5-Diméthylmorpholinyl]-2-(dipropylamino)pyrido[3,4-d]pyrimidin-4(3H)-one

En procédant comme dans l'exemple 8 stade B mais en remplaçant l'ammoniac gazeux par 0,002 mole de di *n*-propylamine, on obtient le produit du titre.
Point de fusion: 195°C

### Exemple 11 :

### 8-[(3α,5β-3,5-Diméthylmorpholinyl]-2-(dipropylamino)pyrido[3,4-d]pyrimidin-4(3H)-one

En procédant comme dans l'exemple 9 et en remplaçant l'ammoniac gazeux par 0,002 mole de di *n*-popylamine, on obtient le produit du titre.
Point de fusion : 173°C

### Exemple 12 :

### 8-[(3α,5α)-3,5-Diméthylmorpholinyl]-2-(4-morpholinyl)pyrido[3,4-d]pyrimidin-4(3H)-one

En procédant comme dans l'exemple 8 stade B mais en remplaçant l'ammoniac gazeux par 0,002 mole de morpholine, on obtient le produit du titre.
Point de fusion : 275°C

### Exemple 13 :

### 2-Amino-8-(1-azépanyl)-6-(4-morpholinyl)pyrido[3,4-d]pyrimidin-4(3H)-one

En procédant comme dans l'exemple 6, mais en remplaçant la di *n*-propylamine par l'ammoniac gazeux, on obtient le produit du titre.
Sublimation à 260°C

### Exemple 14 :

### 8-Chloro-2-(dipropylamino)pyrido[3,4-d]pyrimidin-4(3H)-one

En procédant comme dans l'exemple 1 et en remplaçant la 3-amino-2-azocanopyridine par la 3-amino-2-chloropyridine, on obtient le produit du titre.
Point de fusion : 180°C

### Exemple 15 :

### 2-(Dipropylamino)-8-(1-pyrrolidinyl)pyrido[3,4-d]pyrimidin-4(3H)-one

En procédant comme dans l'exemple 1 et en remplaçant la 3-amino-2-azocano pyridine par la 3-amino-2-(1-pyrrolidinyl)pyridine, on obtient le produit du titre.
Point de fusion : 220°C

### Exemple 16 :

### 8-(3,4-Diméthoxyphényl)-2-(dipropylamino)pyrido[3,4-d]pyrimidin-4(3H)-one

En procédant comme dans l'exemple 1 et en remplaçant la 3-amino-2-azocano pyridine par la 3-amino-2-(3,4-diméthoxyphényl)pyridine, on obtient le produit du titre.
Point de fusion : 202°C

### ETUDE PHARMACOLOGIQUE

### Exemple A : Screening sur un panel de Kinases

En utilisant des techniques classiques de screening mettant en oeuvre les Kinases commercialement disponibles, les produits de l'invention ont montré d'intéressantes propriétés :
◆ activatrices de certaines Kinases pour plusieurs produits de l'invention.
◆ inhibitrices d'autres Kinases pour plusieurs produits de l'invention.
◆ potentialisatrices d'activateurs ou d'inhibiteurs de Kinases pour d'autres produits.

### Exemple B : Activité hypolipémiante

Les produits de l'invention ont été testés *in vivo* chez la souris obèse ob/ob, utilisée comme modèle d'insulinorésistance associée à l'obésité. A titre d'exemple, le composé de l'Exemple 6 baisse significativement les triglycérides à 125 mg/kg per os, alors qu'avec la metformine, la même diminution est obtenue à 250 mg/kg per os.
Dans ce modèle, les composés de l'invention se sont donc révélés être de puissants hypolipémiants.

### Exemple C : Etude de la toxicité aiguë

La toxicité aiguë a été appréciée après administration orale à des lots de 8 souris (26 ± 6 grammes) de doses croissantes de produit à étudier. Les animaux ont été observés à intervalles réguliers au cours de la première journée et quotidiennement pendant les deux semaines suivant le traitement.
Il apparaît que les composés de l'invention sont totalement atoxiques.

### Exemple D : Composition pharmaceutique

Formule de préparation pour 1000 comprimés dosés à 5 mg

| | |
|---|---|
| Composé de l'exemple 8 | 5 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I), 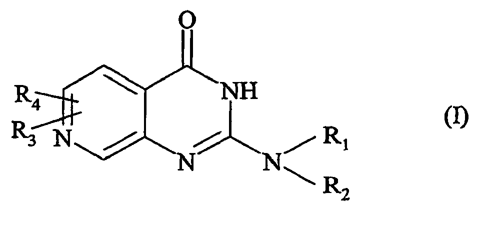 dans laquelle :
➢ R₁ et R₂, identiques ou différents, représentent un atome d'hydrogène, un groupement alkyle ou forment ensemble avec l'atome d'azote qui les porte un hétérocycle,
➢ R₃ représente un atome d'halogène, un groupement alkoxy, un groupement aryle éventuellement substitué, ou un groupement NR'₁R'₂ avec R'₁ et R'₂, identiques ou différents, représentant un atome d'hydrogène, un groupement alkyle ou formant ensemble avec l'atome d'azote qui les porte un hétérocycle,
➢ R₄ représente un atome d'hydrogène ou un groupement N R"₁ R"₂ avec R"₁ et R"₂, identiques ou différents, représentant un atome d'hydrogène, un groupement alkyle ou formant ensemble avec l'atome d'azote qui les porte un hétérocycle,
leur énantiomères, diastéréoisomères, tautomères ainsi que leurs sels d'addition à un acide ou à une base pharmaceutiquement acceptable,
étant entendu que :
- le terme alkyle désigne une chaîne hydrocarbonée, linéaire ou ramifiée, comprenant de 1 à 8 atomes de carbone,
- le terme alkoxy désigne un groupement alkyle oxy dont la chaîne alkyle, linéaire ou ramifiée, contient de 1 à 8 atomes de carbone,
- le terme aryle désigne un groupement phényle ou naphthyle,
- le terme hétérocycle désigne un système mono ou bicyclique, comprenant de 5 à 11 atomes de carbone, et pouvant contenir outre l'atome d'azote auquel sont liés R₁ R₂, R'₁, R'₂, ou R"₁ R"₂ un ou deux autres hétéroatomes choisis parmi oxygène, soufre et azote, ce système hétérocyclique pouvant être substitué par un, deux, ou trois groupements alkyle,
- le terme substitué affectant le groupement aryle signifie que les groupements phényle ou naphthyle sont substitués par un, deux, ou trois groupements, identiques ou différents, choisis parmi les atomes d'halogène, les groupements alkyle, alkoxy, polyhalogénoalkyle, et hydroxy, étant entendu que par polyhalogénoalkyle on entend une chaîne carbonée, linéaire ou ramifiée, contenant de 1 à 3 atomes de carbone et de 1 à 7 atomes d'halogène.

2. Composés de formule (I') selon la revendication 1, 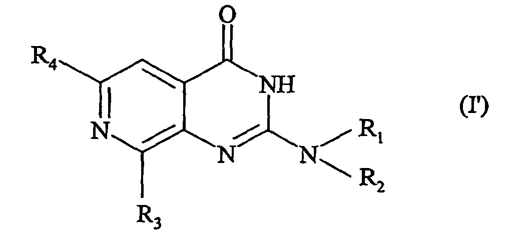 leurs énantiomères, diastéréoisomères, tautomères ainsi que leurs sels d'addition à un acide ou à une base pharmaceutiquement acceptable.

3. Composés de formule (I) selon la revendication 1, pour lesquels N R₁ R₂ représente un groupement NH₂, un groupement di *n*-propylamine ou encore un groupement morpholine, leurs énantiomères, diastéréoisomères, tautomères ainsi que leurs sels d'addition à un acide ou à une base pharmaceutiquement acceptable.

4. Composés de formule (I') selon la revendication 2, pour lesquels NR₁R₂ représente un groupement NH₂, un groupement di *n*-propylamine ou encore une morpholine, leurs énantiomères, diastéréoisomères, tautomères ainsi que leurs sels d'addition à un acide ou à une base pharmaceutiquement acceptable.

5. Composés de formule (I) selon l'une quelconque des revendications 1 ou 3, pour lesquels R₃ représente un groupement 3,4-diméthoxyphényle, 3,5-diméthylmorpholine, thiomorpholine, azépine, perhydroquinoline, ou pyrrolidine, ou un atome de chlore, leur énantiomères, diastéréoisomères, tautomères ainsi que leurs sels d'addition à un acide ou à une base pharmaceutiquement acceptable.

6. Composés de formule (I') selon l'une quelconque des revendications 2 ou 4, pour lesquels R₃ représente un groupement 3,4-diméthoxyphényle, 3,5-diméthylmorpholine, thiomorpholine, azépine, perhydroquinoline, ou pyrrolidine, ou un atome de chlore, leur énantiomères, diastéréoisomères, tautomères ainsi que leurs sels d'addition à un acide ou à une base pharmaceutiquement acceptable.

7. Composés de formule (I) selon l'une quelconque des revendications 1, 3 ou 5, pour lesquels R₄ représente un atome d'hydrogène, un groupement morpholine, ou azépine, leurs énantiomères, diastéréoisomères, tautomères ainsi que leurs sels d'addition à un acide ou à une base pharmaceutiquement acceptable.

8. Composés de formule (I') selon l'une quelconque des revendications 2, 4 ou 6, pour lesquels R₄ représente un atome d'hydrogène, un groupement morpholine, ou azépine, leurs énantiomères, diastéréoisomères, tautomères ainsi que leurs sels d'addition à un acide pharmaceutiquement acceptable.

9. Composé selon la revendication 1 choisis parmi :
la 2-(dipropylamino)-8-(4-thiomorpholinyl)pyrido[3,4-*d*]pyrimidin-4(3*H*)-one,
la 8-(1-azocanyl)-2-(dipropylamino)pyrido[3,4-*d*]pyrimidin-4(3*H*)-one,
la 8-((4a*α*,8a*α*)-octahydro-1(2*H*)-quinolinyl)-2-(dipropylamino)pyrido[3,4-*d*]pyrimidin-4(3*H*)-one,
1a 8-((4a*β*,8a*α*)-octahydro-1(*2H*)-quinolinyl)-2-(dipropylamino)pyrido[3,4-*d*]pyrimidin-4(3*H*)-one,
la 6,8-di(1-azépanyl)-2-(dipropylamino)pyrido[3,4-*d*]pyrimidin-4(3*H*)-one,
la 8-(1-azépanyl)-2-(dipropylamino)-6-(4-morpholinyl)pyrido[3,4-*d*]pyrimidin-4(3*H*)-one,
la 8-(1-azépanyl)-2,6-di(4-morpholinyl)pyrido[3,4-*d*]pyrimidin-4(3*H*)-one,
la 2-amino-8-[(3*α*,5*β*)-3,5-diméthylmorpholinyl]pyrido[3,4-*d*]pyrimidin-4(3*H*)-one,
la 2-amino-8-[(3*α*,5*α*)-3,5-diméthylmorpholinyl]pyrido[3,4-*d*]pyrimidin-4(3*H*)-one,
la 8-[(3*α*,5*β*)-3,5-diméthylmorpholinyl]-2-(dipropylamino)pyrido[3,4-*d*]pyrimidin-4(3*H*)-one,
la 8-[(3*α*,5*α*)-3,5-diméthylmorpholinyl]-2-(dipropylamino)pyrido[3,4-*d*]pyrimidin-4(3*H*)-one,
la 8-[(3*α*,5*α*)-3,5-diméthylmorpholinyl]-2-(4-morpholinyl)pyrido[3,4-*d*]pyrimidin-4(3*H*)-one,
la 2-amino-8-(1-azépanyl)-6-(4-morpholinyl)pyrido[3,4-*d*]pyrimidin-4(3*H*)-one,
la 8-chloro-2-(dipropylamino)pyrido[3,4-*d*]pyrimidin-4(3*H*)-one,
la 2-(dipropylamino)-8-(1-pyrrolidinyl)pyrido[3,4-*d*]pyrimidin-4(3*H*)-one,
et la 8-(3,4-diméthoxyphényl)-2-(dipropylamino)pyrido[3,4-*d*]pyrimidin-4(3*H*)-one,
leurs tautomères et leurs sels d'addition à un acide pharmaceutiquement acceptable.

10. Procédé de préparation des composés de formule (I) selon la revendication 1 **caractérisé en ce que** l'on utilise comme produit de départ les composés de formule (II) : 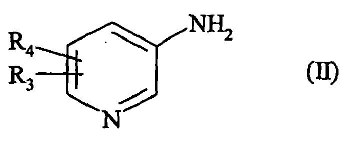 dans laquelle :
R₃ et R₄ ont la même définition que dans la formule (I), que l'on condense avec un dérivé de formule (III) :
S=C=N-C(O)OR₂₀ (III)
avec R₂₀ représentant un groupement alkyle ou arylalkyle,
pour conduire à un dérivé de formule (IV) : 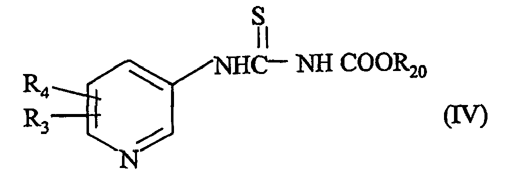
dans laquelle :
R₃, R₄ et R₂₀ ont la même signification que précédemment, dérivé de formule (IV), qui se condense en présence d'un sel métallique avec l'amine (V) :
HN R₁ R₂ (V)
dans laquelle :
R₁ et R₂ ont la même signification que dans la formule (I), pour conduire à un composé de formule (I),
- qui peut-être, le cas échéant, purifié selon une technique classique de purification,
- dont on sépare, le cas échéant, les stéréoisomères selon une technique classique de séparation,
- que l'on transforme, si l'on le souhaite, en ses sels d'addition à un acide ou une base pharmaceutiquement acceptable.

11. Composition pharmaceutique contenant comme principe actif au moins un composé selon quelconque des revendications 1 à 9, seul ou en combinaison avec un ou plusieurs excipients ou véhicules inertes, non toxiques, pharmaceutiquement acceptables.

12. Composition pharmaceutique selon la revendication 11 contenant au moins un principe actif selon l'une quelconque des revendications de 1 à 9 utiles pour la fabrication de médicaments traitant ou prévenant le cancer, le diabète de type II non insulinodépendant, l'obésité, l'hyperlipidémie, l'hypercholestérolémie et leurs complications cardiovasculaires, l'arthrose, l'hypertension artérielle.

13. Composition pharmaceutique selon la revendication 11 contenant au moins un principe actif selon l'une quelconque des revendications de 1 à 9 utiles pour la fabrication de médicaments traitant ou prévenant le diabète de type II et ses complications cardiovasculaires.

14. Composition pharmaceutique selon la revendication 11 contenant au moins un principe actif selon l'une quelconque des revendications de 1 à 9 utiles pour la fabrication de médicaments traitant ou prévenant le cancer.

15. Composition pharmaceutique selon la revendication 11 contenant au moins un principe actif selon l'une quelconque des revendications de 1 à 9 utiles pour la fabrication de médicaments traitant l'arthrose.

16. Composition pharmaceutique selon la revendication 11 contenant au moins un principe actif selon l'une quelconque des revendications de 1 à 9 utiles pour la fabrication de médicaments traitant l'hypertension artérielle.

## Patentansprüche

1. Verbindungen der Formel (I) 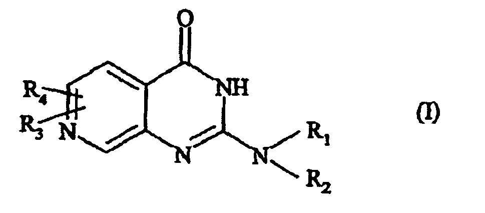 in der:
➢ R₁ und R₂, die gleichartig oder verschieden sind, ein Wasserstoffatom oder eine Alkylgruppe bedeuten oder gemeinsam mit dem sie tragenden Stickstoffatom einen Heterocyclus bilden.
➢ R₃ ein Halogenatom, eine Alkoxygruppe, eine gegebenenfalls substituierte Arylgruppe oder eine Gruppe NR'₁R'₂ darstellt, worin R'₁ und R'₂, die gleichartig oder verschieden sind, ein Wasserstoffatom oder eine Alkylgruppe bedeuten oder gemeinsam mit dem sie tragenden Stickstoffatom einen Heterocyclus bilden,
➢ R₄ ein Wasserstoffatom oder eine Gruppe NR"₁R"₂ bedeutet, worin R"₁ und R"₂, die gleichartig oder verschieden sind, ein Wasserstoffatom oder eine Alkylgruppe bedeuten oder gemeinsam mit dem sie tragenden Stickstoffatom einen Heterocyclus bilden,
deren Enantiomere, Diastereoisomere. Tautomere sowie deren Additionssalze mit einer pharmazeutisch annehmbaren Säure oder Base,
mit der Maßgabe, daß:
- der Begriff Alkyl für eine geradkettige oder verzweigte Kohlenwasserstoffkette steht, die 1 bis 8 Kohlenstoffatome aufweist.
- der Begriff Alkoxy für eine Alkyloxygruppe steht, deren geradkettige oder verzweigte Alkylkette 1 bis 8 Kohlenstoffatome aufweist,
- der Begriff Aryl für ene Phenyl- oder Naphthylgruppe steht,
- der Begriff Heterocyclus für ein mono- oder bicyclisches System steht, welches 5 bis 11 Kohlenstoffatome aufweist und neben dem Stickstoffatom, an das R₁R₂, R'₁R'₂ oder R"₁R"₂ gebunden sind, ein oder zwei weitere Heteroatome ausgewählt aus Sauerstoff, Schwefel und Stickstoff enthalten kann, wobei dieses heterocyclische System durch eine, zwei oder drei Alkylgruppen substituiert sein kann.
- der Begriff substituiert bezüglich der Arylgruppe bedeutet, daß die Phenyl- oder Naphthylgruppen durch eine, zwei oder drei gleichartige oder verschiedenartige Gruppen substituiert sind, ausgewählt aus Halogenatomen, Alkyl-, Alkoxy-, Polyhalogenalkyl- und Hydroxygruppen, mit der Maßgabe, daß man unter einer Polyhalogenalkylgruppe eine geradkettige oder verzweigte Kohlenstoffkette versteht, die 1 bis 3 Kohlenstoffatome und 1 bis 7 Halogenatome aufweist.

2. Verbindungen der Formel (I') nach Anspruch 1 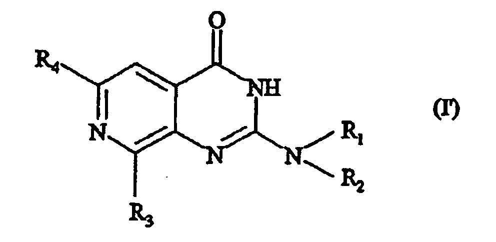 deren Enantiomere, Diastereoisomere, Tautomere sowie deren Additionssalze mit einer pharmazeutisch annehmbaren Säure oder Base.

3. Verbindungen der Formel (I) nach Anspruch 1, worin NR₁R₂ eine Gruppe NH₂, eine Di-*n*-propylamingruppe oder eine Morpholingruppe bedeutet, deren Enantiomere, Diastereoisomere, Tautomere sowie deren Additionssalze mit einer pharmazeutisch annehmbaren Säure oder Base.

4. Verbindungen der Formel (I') nach Anspruch 1, worin NR₁R₂ eine Gruppe NH₂, eine Di-*n*-propylamingruppe oder eine Morpholingruppe bedeutet, deren Enantiomere, Diastereoisomere, Tautomere sowie deren Additionssalze mit einer pharmazeutisch annehmbaren Säure oder Base.

5. Verbindungen der Formel (I) nach einem der Ansprüche 1 bis 3, worin R₃ eine 3,4-Dimethoxyphenyl-, 3,5-Dimethylmorpholin-, Thiomorpholin-, Azepin-, Perhydrochinolin- oder Pyrrolidin-gruppe oder ein Chloratom bedeutet, deren Enantiomere, Diastereoisomere, Tautomere sowie deren Additionssalze mit einer pharmazeutisch annehmbaren Säure oder Base.

6. Verbindungen der Formel (I') nach einem der Ansprüche 2 oder 4, worin R₃ eine 3,4-Dimethoxyphenyl-, 3,5-Dimethylmorpholin-, Thiomorpholin-, Azepin-, Perhydrochinolin- oder Pyrrolidin-gruppe oder ein Chloratom bedeutet, deren Enantiomere, Diastereoisomere, Tautomere sowie deren Additionssalze mit einer pharmazeutisch annehmbaren Säure oder Base.

7. Verbindungen der Formel (I) nach einem der Ansprüche 1, 3 oder 5, worin R₄ ein Wasserstoffatom, eine Morpholin- oder Azepin-gruppe bedeutet, deren Enantiomere, Diastereoisomere, Tautomere sowie deren Additionssalze mit einer pharmazeutisch annehmbaren Säure oder Base.

8. Verbindungen der Formel (I') nach einem der Ansprüche 2, 4 oder 6, worin R₄ ein Wasserstoffatom, eine Morpholin- oder Azepin-gruppe bedeutet, deren Enantiomere, Diastereoisomere, Tautomere sowie deren Additionssalze mit einer pharmazeutisch annehmbaren Säure oder Base.

9. Verbindung nach Anspruch 1, ausgewählt aus:
2-(Dipropylamino)-8-(4-thiomorpholinyl)-pyrido[3,4-*d*]pyrimidin-4(3*H*)-on,
8-(1-Azocanyl)-2-(dipropylamino)-pyrido[3, 4-*d*]pyrimidon-4(3*H*)-on,
8-((4a*α*,8a*α*)-Octahydro-1(2*H*)-chinolinyl)-2-(dipropylamino)-pyrido[3,4-*d*]pyrimidin-4(3*H*)-on,
8-((4a*β*,8a*α*)-Octahydro-1(2*H*)-chinolinyl)-2-(dipropylamino)-pyrido[3,4-*d*]pyrimidin-4(3*H*)-on.
6,8-Di(1-azepanyl)-2-(dipropylamino)-pyrido[3,4-*d*]pyrimidin-4(3*H*)-on,
8-(1-Azepanyl)-2-(dipropylamino)-6-(4-morpholinyl)-pyrido[3,4-*d*]pyrimidin-4(3*H*)-on,
8-(1-Azepanyl)-2,6-di-(4-morpholinyl)-pyrido[3,4-*d*]pyrimidin-4(3*H*)-on,
2-Amino-8-[(3*α*, 5*β*)-3,5-dimethylmorpholinyl]-pyrido[3,4-*d*]pyrimidin-4(3*H*)-on,
2-Amino-8-[(3*α*,5*α*)-3,5-dimethylmorpholinyl]-pyrido[3,4-*d*]pyrimidin-4(3*H*)-on,
8-[(3*α*,5*β*)-3,5-Dimethylmorpholinyl]-2-(dipropylamino)-pyrido[3,4-*d*]pyrimidin-4(3*H*)-on,
8-[(3*α*,5*α*)-3,5-Dimethylmorpholinyl]-2-(dipropylamino)-pyrido[3,4-*d*]pyrimidin-4(3*H*)-on,
8-[(3*α*,5*α*)-3,5-Dimethylmorpholinyl]-2-(4-morpholinyl)-pyrido[3,4-*d*]pyrimidin-4(3*H*)-on,
2-Amino-8-(1-azepanyl)-6-(4-morpholinyl)-pyrido[3,4-*d*]pyrimidin-4(3*H*)-on,
8-Chlor-2-(dipropylamino)-pyrido[3,4-*d*]pyrimidin-4(3*H*)-on,
2-(Dipropylamino)-8-(1-pyrrolidinyl)-pyrido[3,4-*d*]pyrimidin-4(3*H*)-on und
8-(3,4-Dimethoxyphenyl)-2-(dipropylamino)-pyrido[3,4-*d*]pyrimidin-4(3*H*)-on,
deren Tautomere sowie deren Additionssalze mit einer pharmazeutisch annehmbaren Säure.

10. Verfahren zur Herstellung der Verbindungen der Formel (I) nach Anspruch 1, **dadurch gekennzeichnet, daß** man als Ausgangsprodukt die Verbindungen der Formel (II) verwendet: 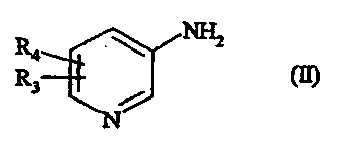 in der:
R₃ und R₄ die bezüglich der Formel (I) angegebenen Bedeutungen besitzen, welche man mit einem Derivat der Formel (III) kondensiert:
S = C = N - C(O)OR₂₀ (III)
worin R₂₀ eine Alkyl- oder Arylalkylgruppe bedeutet,
zur Bildung eines Derivats der Formel (IV): 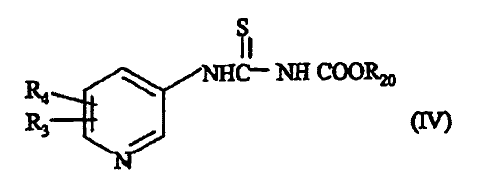
in der:
R₃, R₄ und R₂₀ die oben angegebenen Bedeutungen besitzen, welches Derivat der Formel (IV) man in Gegenwart eines Metallsalzes mit dem Amin (V) kondensiert:
HNR₁R₂ (V)
in der:
R₁ und R₂ die bezüglich der Formel (I) angegebenen Bedeutungen besitzen, zur Bildung einer Verbindung der Formel (I),
- welche man gegebenenfalls mit Hilfe einer klassischen Reinigungsmethode reinigt,
- welche man gegebenenfalls mit Hilfe einer klassischen Trennmethode in ihre Stereoisomeren auftrennt und
- welche man gewünschtenfalls in ihre Additionssalze mit einer pharmazeutisch annehmbaren Säure oder Base überführt.

11. Pharmazeutische Zubereitung enthaltend als Wirkstoff mindestens eine Verbindung nach einem der Ansprüche 1 bis 9 allein oder in Kombination mit einem oder mehreren inerten, nichttoxischen, pharmazeutisch annehmbaren Trägermaterialien oder Hilfsstoffen.

12. Pharmazeutische Zubereitung nach Anspruch 11, enthaltend mindestens einen Wirkstoff nach einem der Ansprüche 1 bis 9, nützlich für die Herstellung von Arzneimitteln zur Behandlung oder Vorbeugung von Krebs, des nicht insulinabhängigen Diabetes Typ II, der Fettsucht, der Hyperlipidämie, der Hypercholesterinämie und ihren kardiovaskulären Komplikationen, der Arthrose und der arteriellen Hypertension.

13. Pharmazeutische Zubereitung nach Anspruch 11, enthaltend mindestens einen Wirkstoff nach einem der Ansprüche 1 bis 9, nützlich für die Herstellung von Arzneimitteln zur Behandlung oder Vorbeugung des Diabetes Typ II und seiner kardiovaskulären Komplikationen.

14. Pharmazeutische Zubereitung nach Anspruch 11, enthaltend mindestens einen Wirkstoff nach einem der Ansprüche 1 bis 9, nützlich für die Herstellung von Arzneimitteln zur Behandlung oder Vorbeugung von Krebs.

15. Pharmazeutische Zubereitung nach Anspruch 11, enthaltend mindestens einen Wirkstoff nach einem der Ansprüche 1 bis 9, nützlich für die Herstellung von Arzneimitteln zur Behandlung der Arthrose.

16. Pharmazeutische Zubereitung nach Anspruch 11, enthaltend mindestens einen Wirkstoff nach einem der Ansprüche 1 bis 9, nützlich für die Herstellung von Arzneimitteln zur Behandlung der arteriellen Hypertension.

## Claims

1. Compounds of formula (I), 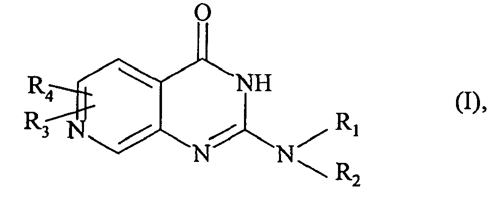 wherein :
➢ R₁ and R₂, which are the same or different, represent a hydrogen atom or an alkyl group or together with the nitrogen atom carrying them form a heterocycle,
➢ R₃ represents a halogen atom, an alkoxy group, an optionally substituted aryl group or a group NR'₁R'₂ wherein R'₁ and R'₂, which are the same or different, represent a hydrogen atom or an alkyl group or together with the nitrogen atom carrying them form a heterocycle,
➢ R₄ represents a hydrogen atom or a group NR"₁R"₂ wherein R"₁ and R"₂, which are the same or different, represent a hydrogen atom or an alkyl group or together with the nitrogen atom carrying them form a heterocycle,
their enantiomers, diastereoisomers, tautomers and also addition salts thereof with a pharmaceutically acceptable acid or base,
it being understood that :
- the term "alkyl" denotes a linear or branched hydrocarbon chain containing from 1 to 8 carbon atoms,
- the term "alkoxy" denotes an alkyl-oxy group wherein the alkyl chain is linear or branched and contains from 1 to 8 carbon atoms,
- the term "aryl" denotes a phenyl or naphthyl group,
- the term "heterocycle" denotes a mono- or bi-cyclic system which contains from 5 to 11 carbon atoms and which may contain, in addition to the nitrogen atom to which R₁R₂, R'₁R'₂ or R"₁R"₂ are bonded, one or two further hetero atoms selected from oxygen, sulphur and nitrogen, it being possible for the heterocyclic system to be substituted by one, two or three alkyl groups,
- the term "substituted" associated with an aryl group indicates that the phenyl or naphthyl group is substituted by one, two or three identical or different groups selected from halogen atoms and alkyl, alkoxy, polyhaloalkyl and hydroxy groups, "polyhaloalkyl" being understood to be a linear or branched carbon chain containing from 1 to 3 carbon atoms and from 1 to 7 halogen atoms.

2. Compounds of formula (I') according to claim 1, 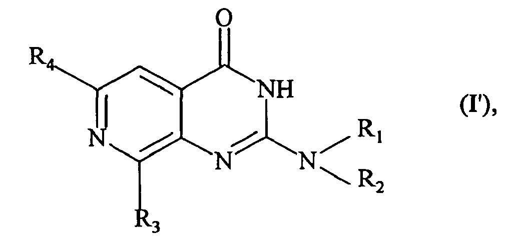 their enantiomers, diastereoisomers, tautomers and also addition salts thereof with a pharmaceutically acceptable acid or base.

3. Compounds of formula (I) according to claim 1, wherein NR₁R₂ represents an NH₂ group, a di-*n*-propylamine group or also a morpholine group, their enantiomers, diastereoisomers, tautomers and also addition salts thereof with a pharmaceutically acceptable acid or base.

4. Compounds of formula (I') according to claim 2, wherein NR₁R₂ represents an NH₂ group, a di-*n*-propylamine group or also a morpholine group, their enantiomers, diastereoisomers, tautomers and also addition salts thereof with a pharmaceutically acceptable acid or base.

5. Compounds of formula (I) according to either claim 1 or claim 3, wherein R₃ represents a 3,4-dimethoxyphenyl, 3,5-dimethylmorpholine, thiomorpholine, azepine, perhydroquinoline or pyrrolidine group or a chlorine atom, their enantiomers, diastereoisomers, tautomers and also addition salts thereof with a pharmaceutically acceptable acid or base.

6. Compounds of formula (I') according to either claim 2 or claim 4, wherein R₃ represents a 3,4-dimethoxyphenyl, 3,5-dimethylmorpholine, thiomorpholine, azepine, perhydroquinoline or pyrrolidine group or a chlorine atom, their enantiomers, diastereoisomers, tautomers and also addition salts thereof with a pharmaceutically acceptable acid or base.

7. Compounds of formula (I) according to any one of claims 1, 3 and 5, wherein R₄ represents a hydrogen atom or a morpholine or azepine group, their enantiomers, diastereoisomers, tautomers and also addition salts thereof with a pharmaceutically acceptable acid or base.

8. Compounds of formula (I') according to any one of claims 2, 4 and 6, wherein R₄ represents a hydrogen atom or a morpholine or azepine group, their enantiomers, diastereoisomers, tautomers and also addition salts thereof with a pharmaceutically acceptable acid.

9. Compound according to claim 1 selected from :
2-(dipropylamino)-8-(4-thiomorpholinyl)pyrido[3,4-*d*]pyrimidin-4(3*H*)-one,
8-(1-azocanyl)-2-(dipropylamino)pyrido[3,4-*d*]pyrimidin-4(3*H*)-one,
8-((4a*α*,8a*α*)-octahydro-1(2*H*)-quinolyl)-2-(dipropylamino)pyrido[3,4-*d*]pyrimidin-4(3*H*)-one,
8-((4a*β*,8a*α*)-octahydro-1(2*H*)-quinolyl)-2-(dipropylamino)pyrido[3,4-*d*]pyrimidin-4(3*H*)-one,
6,8-di(1-azepanyl)-2-(dipropylamino)pyrido[3,4-*d*]lpyrimidin-4(3*H*)-one,
8-(1-azepanyl)-2-(dipropylamino)-6-(4-morpholinyl)pyrido[3,4-*d*]pyrimidin-4(3*H*)-one,
8-(1-azepanyl)-2,6-di(4-morpholinyl)pyrido[3,4-d]pyrimidin-4(3*H*)-one,
2-amino-8-[(3*α*,5*β*)-3,5-dimethylmorpholinyl]pyrido[3,4-*d*]pyrimidin-4(3*H*)-one,
2-amino-8-[(3*α*,5*α*)-3,5-dimethylmorpholinyl]pyrido[3,4-*d*]pyrimidin-4(3*H*)-one,
8-[(3*α*,5*β*)-3,5-dimethylmorpholinyl]-2-(dipropylamino)pyrido[3,4-*d*]pyrimidin-4(3*H*)-one,
8-[(3*α*,5α)-3,5-dimethylmorpholinyl]-2-(dipropylamino)pyrido[3,4-*d*]pyrimidin-4(3*H*)-one,
8-[(3*α*,5*α*)-3,5-dimethylmorpholinyl]-2-(4-morpholinyl)pyrido[3,4-*d*]pyrimidin-4(3*H*)-one,
2-amino-8-(1-azepanyl)-6-(4-morpholinyl)pyrido[3,4-*d*]pyrimidin-4(3*H*)-one,
8-chloro-2-(dipropylamino)pyrido[3,4-*d*]pyrimidin-4(3*H*)-one,
2-(dipropylamino)-8-(1-pyrrolidinyl)pyrido[3,4-*d*]pyrimidin-4(3*H*)-one,
and 8-(3,4-dimethoxyphenyl)-2-(dipropylamino)pyrido[3,4-*d*]pyrimidin-4(3*H*)-one,
their tautomers and also addition salts thereof with a pharmaceutically acceptable acid.

10. Process for the preparation of compounds of formula (I) according to claim 1, **characterised in that** there is used as starting material a compound of formula (II) : 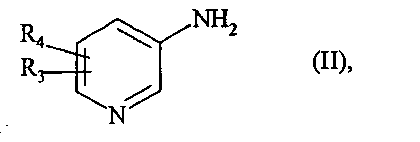 wherein :
R₃ and R₄ are as defined for formula (I), which is condensed with a compound of formula (III) :
S = C = N - C (O) OR₂₀ (III),
wherein R₂₀ represents an alkyl or aryl-alkyl group,
to yield a compound of formula (IV) : 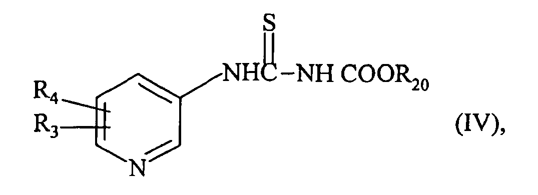
wherein :
R₃, R₄ and R₂₀ are as defined hereinbefore, which compound of formula (IV) is condensed in the presence of a metallic salt with the amine (V) :
HN R₁ R₂ (V),
wherein :
R₁ and R₂ are as defined for formula (I), to yield a compound of formula (I),
- which may be, where appropriate, purified according to a conventional purification method,
- which is separated, where applicable, into its stereoisomers according to a conventional separation technique,
- which is converted, if desired, into its addition salts with a pharmaceutically acceptable acid or base.

11. Pharmaceutical composition comprising as active ingredient at least one compound according to any one of claims 1 to 9, alone or in combination with one or more pharmaceutically acceptable, inert, non-toxic excipients or carriers.

12. Pharmaceutical composition according to claim 11, comprising at least one active ingredient according to any one of claims 1 to 9, for use in producing medicaments treating or preventing cancer, non-insulin-dependent, type II diabetes, obesity, hyperlipidaemia, hypercholesterolaemia and cardiovascular complications thereof, arthrosis, arterial hypertension.

13. Pharmaceutical composition according to claim 11, comprising at least one active ingredient according to any one of claims 1 to 9, for use in producing medicaments treating or preventing type II diabetes and cardiovascular complications thereof.

14. Pharmaceutical composition according to claim 11, comprising at least one active ingredient according to any one of claims 1 to 9, for use in producing medicaments treating or preventing cancer.

15. Pharmaceutical composition according to claim 11, comprising at least one active ingredient according to any one of claims 1 to 9, for use in producing medicaments treating arthrosis.

16. Pharmaceutical composition according to claim 11, comprising at least one active ingredient according to any one of claims 1 to 9, for use in producing medicaments treating arterial hypertension.
